# EUROPÄISCHE PATENTSCHRIFT

(11) **EP 2 531 563 B1**
(45) Veröffentlichungstag und Bekanntmachung des Hinweises auf die Patenterteilung: **09.11.2016**
(21) Anmeldenummer: 11701955.4
(22) Anmeldetag: 02.02.2011
(51) Int. Cl.: C09C 1/00

(54) **EFFEKTPIGMENTE**
EFFECT PIGMENTS
PIGMENTS À EFFETS

(30) Priorität: 04.02.2010 EP 10001163
(43) Veröffentlichungstag der Anmeldung: 12.12.2012
(73) Patentinhaber: Merck Patent GmbH, 64293 Darmstadt (DE)
(72) Erfinder: MELSON, Sabine, 64367 Muehltal (DE); WILHELM, Volker, 64563 Lorsch (DE); SCHOENEFELD, Ulrich, 64404 Bickenbach (DE)
(86) Internationale Anmeldenummer: PCT/EP2011/000464
(87) Internationale Veröffentlichungsnummer: WO 2011/095326

(56) Entgegenhaltungen:
- WO-A1-94/01498
- WO-A1-2004/104110
- DE-A1- 19 639 783

## Beschreibung

Die vorliegende Erfindung betrifft Effektpigmente auf der Basis von unbeschichteten oder beschichteten plättchenförmigen Substraten mit einer äußeren Beschichtung wie sie in Anspruch 1 definiert sind sowie deren Verwendung, u.a. in Farben, Lacken, Druckfarben, Kunststoffen und kosmetischen Formulierungen. Pigmente enthaltend eine oder mehrere Schichten mit einem Oxid und/oder Hydroxid einer Übergangsmetallverbindung, wie z. B. TiO₂, Fe₂O₃, etc. oder Mischungen der Oxide, wie sie z.B. in WO 2008/048922, WO 2006/0181096, EP 0882099 und EP 1422268 beschrieben sind, werden als Glanz- oder Effektpigmente in vielen Bereichen der Technik, insbesondere in der dekorativen Beschichtung, in Kunststoffen, Farben, Lacken, Druckfarben sowie in kosmetischen Formulierungen, eingesetzt. Die Anwendungsmedien enthalten zur Verbesserung ihrer Eigenschaften in der Regel eine Reihe von Zusatzstoffen, wie z. B. Weichmacher, Füllstoffe, Stabilisatoren, Alterungsschutzmittel, Gleit- und Trennmittel, Antistatika und Farbmittel. Pigmente werden im Innen und Außenbereich eingesetzt. Gerade die Außenanwendung stellt hohe Anforderungen an ein Pigment. Hier treten verschiedene Faktoren, wie, Lichteinwirkung, hohe Luftfeuchtigkeit, hohe und niedrige Temperaturen auf, die das Pigment belasten. Insbesondere Kunststoffteile und Lackschichten für Außenanwendungen sind oft über längere Zeit extremen Witterungsverhältnissen und lang anhaltender intensiver Lichteinwirkung ausgesetzt, was zu einer Alterung der Materialien führt. Dies äußert sich in Verfärbungen, Versprödung sowie verminderter mechanischer und chemischer Stabilität. Dabei wird häufig insbesondere zwischen den Effektpigmenten einerseits und Zusatzstoffen im Anwendungsmedium andererseits eine unerwünschte Wechselwirkung beobachtet, die vermutlich darin besteht, dass Übergangsmetallkationen mit den Zusatzstoffen auf organischer Basis reagieren. So beobachtet man häufig in Kunststoffen, dass die Stabilisator- und/oder Alterungsmittelmoleküle zu der Oberfläche der Pigmentteilchen diffundieren und dort zu einer Vergilbungsreaktion führen, die vielfach auch im Dunkeln abläuft, insbesondere wenn die Kunststoffe phenolische Komponenten als Antioxidantien, Thermostabilisatoren oder UV-Stabilisatoren enthalten.

Um diese Alterungsprozesse zu hemmen, werden Formulierungen für Außenanwendungen Stabilisatoren, z. B. UV-Licht absorbierende Stoffe, zugesetzt. Zusätzlich können die Pigmente mit weiteren Schichten, sogenannten Nachbeschichtungen, versehen werden. So beschreibt die EP 0 644 242 Polyolefinzusammensetzungen mit Titandioxid beschichteten Glimmerteilchen, die mit einer oder mehreren Schichten aus SiO₂ und Al₂O₃ beschichtet und calciniert sind. Darüber hinaus enthält die Zusammensetzung ein Antioxidans, um die Vergilbung zu verhindern. Aus WO 1994/001498 sind TiO₂-Pigmente bekannt, die zur Verhinderung der Vergilbung auf einer bevorzugt calcinierten TiO₂-Schicht mit einer Schicht aus Siliziumoxid und/oder Siliziumoxidhydrat, auf dieser siliziumhaltigen Schicht mit einer Schicht aus Aluminiumoxid und/oder Aluminiumoxidhydrat und auf dieser aluminiumhaltigen Schicht mit einer Schicht aus Zinkoxid und/oder Zinkoxidhydrat versehen sind.

WO2004/104110 offenbart Effektpigmente, die eine kalzinierte Oxidschicht besitzen, welche zwingend Vanadium enthält und beispielsweise TiO₂, Al₂O₃, ZnO und SiO₂ enthalten kann.

Auch enthalten die Nachbeschichtungen oft organische Anteile. Die organischen Anteile werden vorzugsweise zum Schutz gegen Feuchtigkeit verwendet. Wird ein solches nachbeschichtetes Pigment dem Prozess der Kunststoffverarbeitung zugeführt, wird das Pigment teilweise mit bis zu 300°C belastet. Diese Temperatur schädigt den organischen Teil, so dass z.B. Vergilbung schon bei der Verarbeitung auftritt oder keine ausreichende Beständigkeit gegen UV-Bestrahlung gegeben ist.

Die derzeitigen Lösungen decken Teile der Anforderungen an Effektpigmente ab, nicht aber die Gesamtheit der Anforderungen. Die bekannten Methoden zur Stabilisierung von Effektpigmenten erfordern zudem alle zusätzliche Verfahrensschritte um die erforderlichen Nachbeschichtungen aufzubringen. Es besteht somit Bedarf an weiteren Verbesserungen und es bestand die Aufgabe, Effektpigmente bereit zustellen, die lichtbeständig sind, ohne die genannten Nachteile aufzuweisen.

Überraschenderweise wurde nun gefunden, dass diese Aufgabe durch die erfindungsgemäßen Effektpigmente gelöst wird. Gegenstand der vorliegenden Erfindung sind daher Effektpigmente auf der Basis unbeschichteter, plättchenförmiger Substrate oder mit ein oder mehreren Metalloxiden beschichteter, plättchenförmiger wie sie in Anspruch 1 definiert sind. Insbesondere besteht die äußere metalloxidhaltige, calcinierte Beschichtung aus TiO₂ und Al₂O₃ oder CaO und SiO₂ und ZnO und/oder mindestens einem Mischoxid dieser Elemente. Besonders bevorzugt besteht die äußere metalloxidhaltige, calcinierte Beschichtung aus TiO₂ und Al₂O₃ und SiO₂ und ZnO und/oder mindestens einem Mischoxid dieser Elemente.

Die erfindungsgemäße äußere metalloxidhaltige, calcinierte Beschichtung, im Folgenden auch äußere Beschichtung genannt, befindet sich auf einer oder mehreren Seiten der Substrate. Bevorzugt umhüllt die erfindungsgemäße äußere Beschichtung die Substrate. Diese äußere Beschichtung weist bevorzugt eine Verteilung der Oxide und/oder Mischoxide auf, bei der die Konzentration von TiO₂ in Substratnähe am höchsten ist und zur Pigmentoberfläche hin abnimmt. Die weiteren erfindungsgemäßen Oxide können dabei gleichmäßig in der Beschichtung verteilt sein. Bevorzugt können abnehmende oder zunehmende Konzentrationen einzelner oder aller Oxide ausgehend von der Pigmentoberfläche vorliegen, wobei die äußere Beschichtung bevorzugt die Al-, Mg-, Ca-, Si- und Zn-Oxide und/oder - mischoxide vorwiegend im äußeren Bereich enthält. Dabei enthält die äußere Beschichtung insbesondere im äußeren Bereich Al₂O₃ (und/oder MgO und/oder CaO), SiO₂ und/oder ZnO. Bevorzugt enthält die äußere Beschichtung Al-, Si- und Zn-oxide und/oder -mischoxide vorwiegend im äußeren Bereich. Bevorzugt ist die Konzentration von SiO₂ und/oder ZnO, insbesondere von ZnO, im äußeren Randbereich der Beschichtung am größten. In einer besonders bevorzugten Ausführungsform der Erfindung weist die äußere Beschichtung die höchste Konzentration von TiO₂, allein oder in Mischung mit Al₂O₃, in Substratnähe auf.

Die unter a) bis d) genannten erfindungswesentlichen Oxide können auch als ein oder mehrere Mischoxide oder als Mischung von Mischoxiden und Oxiden vorliegen. Oxide im Sinne der Erfindung umfassen auch Silikate. Bevorzugt enthalten die Effektpigmente in der äußeren Beschichtung Zink als Oxid oder Mischoxid in den folgenden Mengen, angegeben als ZnO und bezogen auf das Gesamtgewicht des geglühten Pigments: ≥ 0.5 Gew.-%, bevorzugt 0.5- 10 Gew.-%, insbesondere 0.8 - 5 Gew.-% bzw. 1.0 - 5 Gew.-%, ZnO. Bevorzugt enthalten die Effektpigmente in der äußere Beschichtung Aluminium, Magnesium und/oder Calcium als Oxid oder Mischoxid in den folgenden Mengen, angegeben als Al₂O₃, MgO bzw. CaO und bezogen auf das Gesamtgewicht des geglühten Pigments: ≥ 0.1 Gew.-%, bevorzugt 0.1 - 6 Gew.-%, insbesondere 0.5 - 6 Gew.-% bzw. 0.5 - 3 Gew.-%, Al₂O₃, MgO und/oder CaO. Bevorzugt sind Effektpigmente, in denen die Gesamtmenge an Al₂O₃, MgO und/oder CaO in den angegebenen Gewichtprozentbereichen vorliegt. Bevorzugt enthalten die Effektpigmente in der äußeren Beschichtung Silizium als Oxid oder Mischoxid in den folgenden Mengen, angegeben als SiO₂ und bezogen auf das Gesamtgewicht des geglühten Pigments: ≥ 1.5 Gew.-%, bevorzugt 1.5 - 10 Gew.-%, insbesondere 2.5 - 6 Gew.-%, SiO₂. Besonders bevorzugt enthalten die Effektpigmente in der äußeren Beschichtung ≥ 0.5 Gew.-%, ZnO und ≥ 0.1 Gew.-% Al₂O₃ oder CaO, bevorzugt Al₂O₃, und ≥ 1.5, Gew.-% SiO₂, bezogen auf das Gesamtgewicht des geglühten Pigments. Insbesondere bevorzugt enthalten die Effektpigmente in der äußere Beschichtung 0.5- 10 Gew.-% ZnO und 0.1 - 6 Gew.-% Al₂O₃ oder CaO, bevorzugt Al₂O₃, und 1.5 - 10 Gew.-% SiO₂, bezogen auf das Gesamtgewicht des geglühten Pigments. Vor allem Effektpigmente mit einer äußeren Beschichtung mit 0.8 - 5 Gew.-% ZnO und 0.5 - 6 Gew.-% Al₂O₃ und 2.5 - 6 Gew.-% SiO₂, bezogen auf das Gesamtgewicht des geglühten Pigments, sind vorteilhaft. Vor allem Effektpigmente mit einer äußeren Beschichtung mit 1.0 - 5 Gew.-%, insbesondere 1.0 - 2 Gew.-%, ZnO und 0.5 - 3 Gew.-%, insbesondere 1 - 3 Gew.-%, Al₂O₃ und 2.5 - 6 Gew.-%, insbesondere 2.5 - 4 Gew.-%, SiO₂, bezogen auf das Gesamtgewicht des geglühten Pigments, sind besonders vorteilhaft. Das Gewichtsverhältnis von ZnO zu Al₂O₃ ist bevorzugt gleich 0.1 - 10, bevorzugt 0.3 - 10 bzw. 0.4 - 10, vorzugsweise 0.3 - 8, insbesondere 0.3 - 2. Das Gewichtsverhältnis SiO₂/ZnO ist bevorzugt gleich 0.1 - 6, insbesondere 0.2 - 5, bevorzugt 2 - 4.5. In einer Ausführungsform der Erfindung kann das Gewichtsverhältnis SiO₂/ZnO auch gleich 0.4 - 2.0, vorzugsweise 0.8 - 1.2, sein.

Bevorzugt enthalten die Effektpigmente in der äußeren Beschichtung ≥ 20 Gew.-% TiO₂, bezogen auf das Gesamtgewicht des geglühten Pigments, wobei der genaue TiO₂-Gehalt von der gewünschten Interferenzfarbe abhängig ist und die Einstellung dem Fachmann geläufig ist. Weiterhin bevorzugt enthalten die Effektpigmente in der äußeren Beschichtung ≥ 20 Gew.-% TiO₂, ≥ 0.5 Gew.-% ZnO, ≥ 0.1 Gew.-% Al₂O₃ und ≥1.5 Gew.-% SiO₂, bezogen auf das Gesamtgewicht des geglühten Pigments. Vor allem Effektpigmente mit einer äußeren Beschichtung bestehend aus ≥ 20 Gew.-% TiO₂ und 1.0 - 5 Gew.-%, insbesondere 1.0 - 2 Gew.-%, ZnO und 0.5 - 3 Gew.-%, insbesondere 1 - 3 Gew.-%, Al₂O₃, und 2.5 - 6 Gew.-%, insbesondere 2.5 - 4 Gew.-%, SiO₂, bezogen auf das Gesamtgewicht des geglühten Pigments, sind besonders vorteilhaft.

Die äußere Beschichtung des geglühten Pigments hat üblicherweise eine Dicke von >50 nm, bevorzugt >65 nm, insbesondere >80 nm. Die äußere Beschichtung ist dabei dicker als sie es für die Erreichung der gewünschten Interferenzfarbe physikalisch und theoretisch wäre. Dabei beträgt die Dicke der äußeren Beschichtung ≥ 20%, bevorzugt ≥40%, mehr als die physikalische Schichtdicke der entsprechenden Interferenzfarbe. Besonders bevorzugt sind 50 - 80 % mehr. Aber auch äußere Beschichtungen mit einer Dicke von >100% mehr als die physikalische Schichtdicke sind möglich. Grundsätzlich ist die genaue Einstellung und Kontrolle der Schichtdicke im Nanometerbereich für das Erreichen einer bestimmten Interferenzfarbe dem Fachmann bekannt (Gerhard Pfaff, Spezielle Effektpigmente, Vincentzverlag, Abb. 2.25 und Abb. 2.26; Seiten 50-53). Ein Silberweißpigment auf Basis von TiO₂ weist nach physikalischen Gesetzmäßigkeiten eine ca. 50 nm dicke TiO₂-Schicht auf. Die erfindungsgemäße äußere Beschichtung eines Silberweißpigments kann dagegen bis zu 120 nm dick sein. Die erfindungsgemäße äußere Beschichtung eines Interferenz-Gold kann bis zu 150 nm dick sein. Andere Interferenzfarben zeigen entsprechende Schichtdicken. Eine übliche blaue Interferenzschicht ist ca. 120 nm dick, eine erfindungsgemäße äußere Beschichtung kann dagegen um 10-100 nm dicker sein.

Besonders vorteilhaft ist es, wenn die erfindungsgemäßen Effektpigmente eine spezifische Oberfläche von ≤ 4 m²/g, bevorzugt ≤ 3 m²/g, gemessen nach der BET-Methode (DIN ISO 9277: 2003-05) aufweisen. Die spezifischen Oberflächen von erfindungsgemäßen Pigmenten und solchen des Standes der Technik (jeweils bei 750°C geglüht) unterscheiden sich z.B. wie folgt:
a) Glimmer der Fraktion 5-25 µm mit einer geglühten TiO₂-Schicht zeigt eine Oberfläche von 7,9 m²/g.
b) Glimmer der Fraktion 5-25 µm mit einer erfindungsgemäßen geglühten äußeren Beschichtung zeigt eine Oberfläche von 2,4 m²/g.
c) Glimmer 10 - 60 µm mit einer geglühten TiO₂-Schicht zeigt eine Oberfläche von 6.5 m²/g.
d) Glimmer 10 - 60 µm mit einer erfindungsgemäßen geglühten äußeren Beschichtung zeigt beispielsweise eine Oberfläche von 2.0 - 2.4 m²/g auf.

Bevorzugt zeigen die erfindungsgemäßen Effektpigmente eine Reduktion der Oberfläche von 30 - 80 %, bevorzugt ≥ 40 %, insbesondere bevorzugt 40 - 65 %, verglichen mit einem eine geglühte TiO₂-Schicht als äußere Schicht enthaltenden Pigment.

Vor allem Effektpigmente mit einer äußeren Beschichtung bestehend aus ≥ 20 Gew.-% TiO₂ und 1.0 - 5 Gew.-%, insbesondere 1.0 - 2 Gew.-%, ZnO und 0.5 - 3 Gew.-%, insbesondere 1 - 3 Gew.-%, Al₂O₃, und 2.5 - 6 Gew.-%, insbesondere 2.5 -4 Gew.-%, SiO₂, bezogen auf das Gesamtgewicht des geglühten Pigments, einer Dicke der äußeren Beschichtung von ≥ 40%, bevorzugt 50 - 80 %, mehr als die physikalische Schichtdicke der entsprechenden Interferenzfarbe und einer spezifische Oberfläche von ≤ 3 m²/g gemessen nach der BET-Methode sind besonders vorteilhaft.

Geeignete Substrate für die erfindungsgemäßen Effektpigmente sind z. B. alle bekannten beschichteten oder unbeschichteten, plättchenförmigen Substrate, vorzugsweise transparente oder semitransparente Plättchen. Geeignet sind z. B. Schichtsilikate, insbesondere synthetischer oder natürlicher Glimmer, Glasplättchen, Metallplättchen, SiOₓ-Plättchen (x = ≤ 2,0, vorzugsweise ist x = 2), Al₂O₃-Plättchen, TiO₂-Plättchen, synthetische oder natürliche Eisenoxidplättchen, Graphitplättchen, Liquid Crystal Polymers (LCPs), holographische Pigmente, BiOCl-Plättchen oder Gemische der genannten Plättchen. Die Metallplättchen können unter anderem aus Aluminium, Titan, Bronze, Stahl oder Silber bestehen, vorzugsweise aus Aluminium und/oder Titan. Die Metallplättchen können dabei durch entsprechende Behandlung passiviert sein. Bevorzugt sind beschichtete oder unbeschichtete Plättchen aus synthetischen oder natürlichen Glimmer, Glasplättchen, SiO₂-Plättchen und Al₂O₃-Plättchen, insbesondere synthetische oder natürliche Glimmerplättchen und SiO₂-Plättchen. In einer Ausführungsform der Erfindung sind unbeschichtete, synthetische oder natürliche, Glimmerplättchen bevorzugt.

In der Regel haben die plättchenförmigen Substrate eine Dicke zwischen 0,05 und 5 µm, insbesondere zwischen 0,1 und 4,5 µm. Glasplättchen haben vorzugsweise eine Dicke von ≤ 1 µm, insbesondere von ≤ 900 nm und ganz besonders bevorzugt von ≤ 500 nm. Die Größe der Substrate ist an sich nicht kritisch und kann auf den jeweiligen Anwendungszweck abgestimmt werden. Üblicherweise beträgt die Partikelgröße 1 - 350 µm, vorzugsweise 2 - 200 µm, und insbesondere zwischen 5 - 150 µm. In der Regel können sowohl grobe Plättchen mit Partikelgrößen von 10 - 200 µm, vorzugsweise von 40 - 200 µm, insbesondere von 10 - 130 µm, als auch feinen Plättchen mit Partikelgrößen von 1 - 60 µm, vorzugsweise von 5 - 60 µm, insbesondere von 10 - 40 µm, verwendet werden. Bevorzugt können auch Substratgemische bestehen aus Plättchen mit unterschiedlichen Partikelgrößen eingesetzt werden. Besonders bevorzugte Substratgemische bestehen aus groben und feine Plättchen, insbesondere aus S-Glimmer (> 125 µm) und F-Glimmer (< 25 µm). Die Partikelgrößen werden mit dem Fachmann bekannten und handelsüblichen Geräten (z. B. von der Fa. Malvern, Fa. Horiba) mittels Laserbeugung am Pulver oder an Pigmentsuspensionen bestimmt. Die Substrate besitzen vorzugsweise einen Formfaktor (aspect ratio: Durchmesser / Dicke-Verhältnis) von 5 - 750, insbesondere von 10 - 300 und ganz besonders bevorzugt von 20 - 200. Darüber hinaus ist auch die Verwendung anderer Substrate, wie z. B. sphärischer Partikel oder nadelförmiger Substrate, die mit den oben genannten Schichten belegt sein können, möglich.

In einer weiteren Ausführungsform kann das Substrat auf einer oder mehr Seiten mit einer oder mehreren transparenten, semitransparenten und/oder opaken Schichten enthaltend Metalloxide, Metalloxidhydrate, Metallsuboxide, Metalle, Metallfluoride, Metallnitride, Metalloxynitride oder Mischungen dieser Materialien beschichtet sein. Bevorzugt ist das Substrat von diesen Schichten umhüllt. Die Metalloxid-, Metalloxidhydrat-, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8, bevorzugt von ≥ 2,0.) sein. Als Metalloxide und Metalloxidhydrate eignen sich alle dem Fachmann bekannten Metalloxide oder Metalloxidhydrate, wie z. B. Aluminiumoxid, Aluminiumoxidhydrat, Siliziumoxid, Siliziumoxidhydrat, Eisenoxid, Zinnoxid, Ceroxid, Zinkoxid, Zirkoniumoxid, Chromoxid, Titanoxid, insbesondere Titandioxid, in der Rutil- oder Anatas-Modifikation, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt werden Metalloxid-, Metall-, Metallfluorid und/oder Metalloxidhydratschichten und ganz besonders bevorzugt Metalloxid- und/oder Metalloxidhydratschichten auf das Substrat aufgebracht. Insbesondere bevorzugt sind Oxide und/oder Oxidhydrate des Aluminiums, Siliziums, Eisens, Zinns und Titans, insbesondere Titandioxid, in der Rutil- oder Anatas-Modifikation, bevorzugt in der Rutilmodifikation, und Gemischen dieser Verbindungen. Zur Rutilisierung von Titandioxid wird üblicherweise eine Zinndioxidschicht unter einer Titandioxidschicht aufgebracht. So können die erfindungsgemäßen Effektpigmente zur Rutilisierung des in der erfindungswesentlichen äußeren Beschichtung enthaltenen Titandioxids auch eine Zinndioxidschicht zwischen Substrat und äußerer Beschichtung enthalten. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln. Insbesondere bevorzugt sind Schichtpakete aus einer hochbrechenden Schicht (Brechzahl ≥ 2,0) und einer niedrigbrechenden Schicht (Brechzahl < 1.8), wobei auf dem Substrat eines oder mehrere dieser Schichtpakete aufgebracht sein können. Die Reihenfolge der hoch- und niedrigbrechenden Schichten kann dabei an das Substrat angepasst werden, um das Substrat in den Mehrschichtaufbau mit einzubeziehen. In einer weiteren Ausführungsform können die Metalloxid-, Metalloxidhydrat-, Metallsuboxid-, Metall-, Metallfluorid-, Metallnitrid-, Metalloxynitridschichten mit Farbmitteln oder anderen Elementen versetzt oder dotiert sein. Als Farbmittel oder andere Elemente eignen sich beispielsweise organische oder anorganische Farbpigmente wie farbige Metalloxide, z.B. Magnetit, Chromoxid oder Farbpigmente wie z.B. Berliner Blau, Ultramarin, Bismutvanadat, Thenards Blau, oder aber organische Farbpigmente wie z.B. Indigo, Azopigmente, Phthalocyanine oder auch Karminrot oder Elemente wie z.B. Yttrium oder Antimon. Effektpigmente enthaltend diese Schichten zeigen eine hohe Farbenvielfalt in Bezug auf ihre Körperfarbe und können in vielen Fällen eine winkelabhängige Änderung der Farbe (Farbflop) durch Interferenz zeigen.

Die Schichten aus Metalloxiden, -hydroxid und/oder -oxidhydraten werden vorzugsweise nasschemisch aufgebracht, wobei die zur Herstellung von Effektpigmenten entwickelten nasschemischen Beschichtungsverfahren angewendet werden können, die zu einer Umhüllung des Substrats führen. Derartige Verfahren sind z.B. beschrieben in DE 14 67 468, DE 20 09 566, DE 22 14 545, DE 22 15 191, DE 22 44, 298, DE 23 13 331, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen. Beispiele und Ausführungsformen der oben genannten Materialien und Pigmentaufbauten finden sich z.B. auch in den Research Disclosures RD 471001 und RD 472005.

Die Dicke der einzelnen Schichten auf dem Substrat ist, wie dem Fachmann geläufig ist, wesentlich für die optischen Eigenschaften des Pigments. Die Dicke der Metalloxid-, Metalloxidhydrat-, Metallsuboxid-, Metall-, Metallfluorid-, Metallnitrid-, Metalloxynitridschichten oder einer Mischung daraus beträgt üblicherweise 10 bis 1000 nm, bevorzugt 15 bis 800 nm, insbesondere 20 bis 600 nm. Besonders geeignet sind Schichtdicken von 20 bis 200 nm. Die Dicke der Metallschichten beträgt vorzugsweise 4 bis 50 nm.

Auf die erfindungswesentliche äußere metalloxidhaltige, calcinierte Beschichtung wie sie in Anspruch 1 definiert ist kann, falls es für bestimmte Anwendungen wie z. B. spezielle Lacke gewünscht ist, zusätzlich eine organische Beschichtung aufgebracht werden. Durch die organische Beschichtung werden die Oberflächeneigenschaften der calcinierten Oxidschichten positiv beeinflusst. Die mit der organischen Beschichtung versehenen Oberflächen sind hydrophober und weniger polar als die unbehandelten Oxidoberflächen und sind damit von Bindemitteln und organischen Lösungsmitteln besser benetzbar. Daraus resultiert eine bessere Verträglichkeit der erfindungsgemäßen Pigmente mit den bei der Anwendung verwendeten Bindemittelsystemen. Ferner hemmt die organische Beschichtung wegen ihrer sterischen Abschirmung der Pigmentoberfläche die Agglomeration der Pigmentpartikel und verbessert so deren Dispergierbarkeit. Diese organische Beschichtung kann aus Kupplungsreagenzien wie z. B. Organosilanen, - aluminaten, -titanaten und/oder zirkonaten bestehen. Vorzugsweise handelt es sich bei den Kupplungsreagenzien um Organosilane. Beispiele für Organosilane sind Propyltrimethoxysilan, Propyltriethoxysilan, Isobutyltrimethoxysilan, n-Octyltrimethoxysilan, i-Octyltrimethoxysilan, n-Octyltriethoxysilan, n-Decyltrimethoxysilan, Dodecyltrimethoxysilan, Hexadecyltrimethoxysilan, Vinyltrimethoxysilan, vorzugsweise n-Octyltrimethoxysilan und n-Octyltriethoxysilan. Als oligomere, alkoholfreie Organosilanhydrolysate eignen sich unter anderem die unter dem Handelsnamen Dynasylan^{®} Hydrosil von der Fa. Evonik Industries vertriebenen Produkte, wie z. B. Dynasylan^{®} Hydrosil 2926, Dynasylan^{®} Hydrosil 2909, Dynasylan^{®} Hydrosil 2907, Dynasylan^{®} Hydrosil 2781, Dynasylan^{®} Hydrosil 2776, Dynasylan^{®} Hydrosil 2627. Darüber hinaus eignet sich oligomeres Vinylsilan als auch Aminosilanhydrolysat als organische Beschichtung. Funktionalisierte Organosilane sind beispielsweise 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan, 1,3-bis(3-glycidoxypropyl)-1,1,3,3,-tetramethyldisiloxan, Ureidopropyltriethoxysilan, bevorzugt sind 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan. Beispiele für polymere Silansysteme sind in WO 98/13426 beschrieben und werden z. B. von der Fa. Evonik Industries unter dem Warenzeichen Dynasylan^{®} Hydrosil vertrieben. Die Menge der organischen Beschichtung kann zwischen 0.2 und 5 Gew.-%, bezogen auf das Pigment, vorzugsweise 0.5 bis 2 Gew.-% betragen.

Ein weiterer Gegenstand der Erfindung ist ein Verfahren zur Herstellung der erfindungsgemäßen Effektpigmente. Die Herstellung der erfindungsgemäßen äußeren Beschichtung der plättchenförmigen Substrate erfolgt bevorzugt nasschemisch. Anschließend werden die Effektpigmente calciniert. Diese Verfahren sind dem Fachmann geläufig. Bei nass-chemischer Aufbringung erfolgt ein Abscheiden der entsprechenden Oxiden, Hydroxiden und/oder Oxidhydraten auf den Substraten, wobei die Substrate bevorzugt umhüllt werden. Dazu werden die plättchenförmigen Substrate in einem Lösungsmittel, vorzugsweise Wasser, suspendiert und mit Lösungen der Metallsalze von a) Ti und b)Al, Mg oder Ca und c) Si und d) Zn versetzt. Hierbei werden die Oxide, Hydroxide und/oder Oxidhydrate auf die Substrate aufgefällt, wobei eine Umhüllung der Substrate erfolgt. Zur Rutilisierung des in der erfindungsgemäßen äußeren Beschichtung enthaltenen Titandioxid kann üblicherweise eine Zinndioxidschicht zwischen Substrat und erfindungsgemäßer äußerer Beschichtung aufgebracht werden. Als Ausgangsverbindungen für die auszufällenden Oxide, Hydroxide und/oder Oxidhydrate eignen sich die entsprechenden Halogenide, Nitrate und/oder Sulfate, vorzugsweise werden die entsprechenden Halogenide und/oder Nitrate eingesetzt. Die Oxide, Hydroxide und/oder Oxidhydrate des Siliziums werden bevorzugt mittels eines Silans, insbesondere TEOS (Tetraethoxysilan), aufgebracht. Die Metallsalzlösungen werden gleichzeitig oder nacheinander bei einem für die Hydrolyse der Salze geeigneten pH-Wert zugegeben, wobei der pH-Wert so ausgewählt ist, dass die Metalloxide bzw. Hydroxide bzw. Oxidhydrate direkt auf den Substraten abgeschieden werden. Bevorzugt können die Ti- und die AI- (oder Mg- oder Ca-)-Salzlösungen gleichzeitig zudosiert werden, wobei ein Teil der Ti-Salzlösung auch zu Anfang allein zugegeben werden kann. Die Zugabe der Si- und Zn-Salzlösungen kann auch gleichzeitig erfolgen. Es ist aber auch möglich, die Si-Salzlösung zuerst und dann die Zn-Salzlösung oder umgekehrt zuzugeben. Es ist auch möglich die Zn-Salzlösung zuerst zuzugeben, dann Al-Salzlösung zuzugeben und dann die Si-Salzlösung. Bevorzugt können die AI- und die Si-Salzlösungen dann gleichzeitig zudosiert werden. Der pH-Wert wird üblicherweise durch gleichzeitiges Zudosieren einer Base und/oder Säure konstant gehalten. Die Einstellung des für die Fällung des jeweiligen Materials erforderlichen pH-Werts und der Temperatur ist dem Fachmann geläufig.

Nach dem nass-chemischer Aufbringung der erfindungsgemäßen äußeren Beschichtung können die Substanzen als Oxide, Hydroxide und/oder Oxidhydrate vorliegen. Bevorzugt werden auf die Substrate basierend auf den eingesetzten Rohstoffen die folgenden Mengen der unter a) bis d) genannten erfindungswesentlichen Elemente als Oxide, Hydroxide und/oder Oxidhydrate aufgefällt, wobei die Mengen als Oxide angegeben sind und die Gew.-% auf das Substrat bezogen sind: ≥ 1 Gew.-%, bevorzugt 1.5- 12 Gew.-%, insbesondere 2 - 12 Gew.-%, ZnO, ≥ 0.5, bevorzugt 1 - 10 Gew.-%, insbesondere 2 - 5 Gew.-%, Al₂O₃, MgO und/oder CaO oder Mischungen dieser Metalloxide, bevorzugt Al₂O₃, ≥ 2 Gew.-%, bevorzugt 1 - 15 Gew.-%, insbesondere 2.5 - 8 Gew.-%, SiO₂ und ≥ 20 Gew.-%, bevorzugt ≥ 25 Gew.-%, TiO₂, wobei der genaue TiO₂-Gehalt von der gewünschten Interferenzfarbe abhängig ist und die Einstellung dem Fachmann geläufig ist.

Anschließend werden die beschichteten Produkte abgetrennt, gewaschen, getrocknet und calciniert. Die bei der nass-chemischen Aufbringung gebildeten Oxide, Hydroxide und/oder Oxidhydrate werden dadurch in die entsprechenden Oxide und/oder Mischoxide der erfindungsgemäßen äußeren Beschichtung überführt. Die Trocknung kann bei Temperaturen von 50 - 150 °C für üblicherweise ≥ 10 Minuten, ggf. für 6 - 18 Stunden, erfolgen. Die Calcinierung kann bei Temperaturen von 250-1000°C, vorzugsweise bei 500-900°C, für üblicherweise 0.5 - 3 Stunden erfolgen. Durch die Calcinierung werden die ausgefällten Oxide, Hydroxide und/oder Oxidhydrate entwässert, in die entsprechenden Oxide bzw. Mischoxide überführt und verdichtet.

Nach der Calcinierung kann, falls es für spezielle Anwendungen gewünscht ist, auf die äußere metalloxidhaltige, calcinierte Beschichtung eine organische Beschichtung aufgebracht werden. Die Aufbringung der Kupplungsreagenzien erfolgt in Lösung bei Temperaturen oberhalb von 60°C, vorzugsweise oberhalb von 70°C. Als Lösemittel eignen sich organische Lösemittel, Wasser oder Mischungen hieraus, bevorzugt wird Wasser verwendet. Die für die Aufbringung der organischen Beschichtung nötige Reaktionszeit liegt bei mindestens 5 Minuten, vorzugsweise erfolgt sie über einen Zeitraum von 10 bis 90 Minuten, kann aber auch beliebig verlängert werden. Das erhaltene Pigment wird nach für den Fachmann gebräuchlichen Methoden aufgearbeitet und isoliert, z. B. durch Filtration, Trocknung und Siebung.

Zur Herstellung von erfindungsgemäßen Effektpigmenten, die unter der erfindungswesentlichen äußeren Beschichtung metalloxidbeschichtete Substrate aufweisen, kann die erfindungswesentliche äußeren Beschichtung auf metalloxidbeschichtete Substrate, die auch calciniert sein können, aufgebracht werden. Bevorzugt werden solche mehrschichtigen erfindungsgemäßen Effektpigmente aber so hergestellt, dass die im Vorangegangenen genannten Metalloxidschichten, vor allem die bevorzugten Metalloxidschichten, die auf dem Substrat unter der erfindungsgemäßen äußeren Beschichtung liegen, direkt vor dem Aufbringen der erfindungsgemäßen äußeren Beschichtung aufgebracht werden und die Aufarbeitung durch Waschen, Trocknen und Calcinieren in einem Schritt erfolgt. In einer insbesondere bevorzugten Ausführungsform der Erfindung wird die erfindungsgemäße äußere Beschichtung auf unbeschichtete Substrate, insbesondere aus synthetischem oder natürlichem Glimmer, aufgebracht.

Ein Vorteil der erfindungsgemäßen Effektpigmente ist dieses einfache Herstellungsverfahren, das keine Zwischenschritte wie z. B. Trocknung und/oder Calcinierung und Isolierungen von Zwischenprodukten erforderlich macht. Weitere Vorteile der erfindungsgemäßen Effektpigmente sind eine geringe bzw- keine Dunkelvergilbung, eine geringe bzw. keine Vergilbung unter UV-Bestrahlung und Feuchtigkeit mit phenolischen Antioxidantien (z.B. BHT), eine hohe Lichtechtheit und eine hohe Wetterstabilität.

Die erfindungsgemäßen Effektpigmente eignen sich somit insbesondere für Anwendungen im Kunststoffbereich, da sie bevorzugt frei von organischen Nachbeschichtungen und trotzdem bewitterungsstabil, lichtecht, und vergilbungsarm sind und eine geringe Photoaktivität aufweisen. Die Lagerstabilität der erfindungsgemäßen Pigmente kann zudem deutlich erhöht sein. Weiterhin wird bei der Pigmentierung mit den erfindungsgemäßen Effektpigmenten eine geringere Vergilbung im Lack und Kunststoff beobachtet. Aufgrund der verbesserten anwendungstechnischen Eigenschaften eignen sich die hier beschriebenen oberflächenmodifizierten Effektpigmente für eine Vielzahl von Anwendungen. Gegenstand der Erfindung ist damit weiterhin die Verwendung der erfindungsgemäßen Effektpigmente in Farben, Lacken, insbesondere Automobillacken, Industrielacken, Pulverlacken, Druckfarben, Sicherheitsanwendungen, kosmetischen Formulierungen, Kunststoffen, keramischen Materialien, Gläsern, Papier, in Tonern für elektrophotographische Druckverfahren, im Saatgut, in Gewächshausfolien und Zeltplanen, als Absorber bei der Lasermarkierung von Papier und Kunststoffen, als Absorber beim Laserschweißen von Kunststoffen und in kosmetischen Formulierungen. Insbesondere die Verwendung der erfindungsgemäßen Effektpigmente in Lacken, insbesondere Automobillacken, und die Verwendung in Kunststoffen ist bevorzugt. Weiterhin sind die erfindungsgemäßen Pigmente auch zur Herstellung von Pigmentanteigungen mit Wasser, organischen und/oder wässrigen Lösemitteln, Pigmentpräparationen sowie zur Herstellung von Trockenpräparaten, wie z. B. Granulaten, Chips, Pellets, Briketts, etc., geeignet. Die Einarbeitung der Pigmente in die jeweiligen Applikationsmedien kann nach allen dem Fachmann bekannten Verfahren erfolgen.

Bevorzugt eingesetzt werden die erfindungsgemäßen Effektpigmente in Lacken wie z. B. in Autolacken oder Wasserlacken, die aufgrund der besonderen Stabilität der Pigmente für alle Innen- und Außenanwendungen geeignet sind. Bevorzugt können alle dem Fachmann bekannten Kunststoffe und Folien vorteilhaft mit den Effektpigmenten gemäß der vorliegenden Erfindung pigmentiert werden, wobei die Einbindung der Pigmente sowohl rein physikalisch durch Mischung als auch chemisch durch Reaktion entsprechender funktioneller Gruppe in der organischen Beschichtung mit dem Kunststoff erfolgen kann. Die erfindungsgemäßen Effektpigmente eignen sich ebenso zur Verwendung in Abmischungen mit organischen Farbstoffen und/oder Pigmenten, wie z.B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Plättchen auf Basis von Glimmer, Glas, Al₂O₃, Fe₂O₃, SiO₂, etc. Die erfindungsgemäßen Effektpigmente können in jedem Verhältnis mit handelsüblichen Pigmenten und Füllern gemischt werden.

Als Füllstoffe sind z. B. natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen. Sie kann den Anforderungen gemäß z.B. plättchenförmig, sphärisch oder nadelförmig sein.

Die Offenbarungen in den zitierten Literaturstellen gehören hiermit ausdrücklich auch zum Offenbarungsgehalt der vorliegenden Anmeldung. Die folgenden Beispiele erläutern die vorliegende Erfindung näher, ohne den Schutzbereich zu beschränken. Insbesondere sind die in den Beispielen beschriebenen Merkmale, Eigenschaften und Vorteile der den betreffenden Beispielen zugrunde liegenden Verbindungen und Formulierungen auch auf andere nicht im Detail aufgeführte, aber unter den Schutzbereich fallende Stoffe und Verbindungen anwendbar, sofern an anderer Stelle nicht Gegenteiliges gesagt wird. Die Feinjustierung der in den Beispielen genannten Parameter ist dem Fachmann im individuellen Fall ohne weiteres möglich, z.B. durch ein statistisches Versuchsdesign nach Box-Behnken, beschrieben in Statistics for Experimenters, Whiley-Interscience, John Whiley and Sons, New York, 1978. Im Übrigen ist die Erfindung im gesamten beanspruchten Bereich ausführbar und nicht auf die hier genannten Beispiele beschränkt.

### Beispiele

### Beschreibung der Testmethode zur Prüfung der Vergilbung:

Die Vergilbung von Pigmenten entsteht hauptsächlich in Gegenwart von phenolischen Antioxidantien unter UV-Bestrahlung bei hoher Temperatur und Luftfeuchte. Insbesondere kostengünstige PE-Typen enthalten BHT (2,6-Di-tert-butyl-4-hydroxy-toluol) als Antioxidans. Dieses Antioxidans bildet mit TiO₂ gelbe Komplexverbindungen. Pigmentierte mit BHT stabilisierte PE-HD Plättchen werden im Suntest XLS Prüfgerät (Firma Atlas) einer gefilterten Xenonstrahlung ausgesetzt. Die visuell sichtbaren Veränderungen werden unter Anwendung des Graumaßstabs nach DIN EN 20105-A02 bewertet. Verwendet werden spritzgegossene Plättchen aus Hostalen GA 7260 (PE-HD), die 1 % Massenanteil des zu prüfenden Pigments und 0,3% BHT enthalten. BHT wird in Form eines Masterbatches von 1% Butylhydroxytoluol (Merck Schuchardt) in Hostalen GA 7260 zudosiert. Der Probenraum wird mit Wasser geflutet, so dass die Plättchen komplett mit Wasser bedeckt sind. Zur Abmusterung werden die Plättchen kurz aus dem Gerät genommen, abgetrocknet, visuell bewertet. Die Abmusterung erfolgt unter der Tageslichtlampe. Abmusterung nach 250h. Die Beurteilungsskala reicht von 5 (sehr gut) bis 1 (sehr schlecht).

### Beschreibung der Testmethode zur Lichtstabilität:

Von einer "Lichtstabilität von Pigmenten" zu sprechen ist eigentlich nicht sinnvoll, da üblicherweise in der Praxis kein Pigment ohne Bindemittel (Bindemittel, Kunststoff o. ä.) eingesetzt wird. Sobald aber Pigmente zusammen mit Bindemitteln/Kunststoffen verwendet werden, wird keine Lichtstabilität von Pigmenten, sondern vom Gesamtsystem - Pigment + Bindemittel + Hilfsstoffe - bestimmt. Angaben zur "Lichtstabilität von Pigmenten" sind daher immer systemabhängig. Die Lichtstabilität der Pigmenten i. o. genannten Sinne wird in spritzgegossenen PE-HD-Plättchen mit einer Dicke von 1,5 mm und bei einer Pigmentkonzentration von 1 Massenanteil geprüft. Die Belichtung der PE-HD Plättchen erfolgt im Xenotest 150 S (Firma Atlas, Filtersystem: "Belichtung im Freien" 1 UV Filter + 6 IR Filter) vergleichend zum Blaumaßstab (DIN EN ISO 105-B01). Die Bewertung der Farbveränderungen der belichteten gegen die unbelichteten Oberflächen der Spritzlinge erfolgt nach der Grauskala (DIN EN 20105-A02). Als Lichtstabilität wird hier die Stufe des Blaumaßstabs definiert, bei dem eine irreversible Veränderung von 4 oder mehr (d. h. 3, 2...) sowohl im Glanzwinkel (bei nahezu senkrechter Betrachtung) in der Aufsicht, als auch in der Durchsicht eingetreten ist. Änderungen, die unter anderen Betrachtungsrichtungen zu beobachten sind werden hierbei nicht berücksichtigt. Die Beurteilungsskala reicht von 8 (sehr gut) bis 1 (sehr schlecht).

### Beschreibung der Testmethode zur Bewitterungsstabilität

Die Muster werden zur Prüfung der Bewitterung in PMMA nach DIN 11341 über 1000h getestet. Die Proben bestehen aus spritzgegossenem Plexiglas 7N mit 1Gew.-% Pigment und werden auf dem Xenotest Beta+ (Firma Atlas) gemäß DIN 11341 über 1000h bewittert. Die Beurteilungsskala reicht von 5 (sehr gut) bis 1 (sehr schlecht).

### Vorschrift zur Prüfung der Photoaktivität

Der Photoaktivitätstest erlaubt eine Charakterisierung der photokalytischen Wirksamkeit von TiO₂ oder TiO₂-haltigen Pigmenten. Als Prüfmedium dient eine PVC-Mischung, die als Reaktionspartner Pb²+-Salze und Glycerinmonooleat enthält. Während der Prüfung werden unter dem Einfluss des photoaktiven TiO₂ Pb²+-Ionen zu Pb reduziert, was makroskopisch als Vergrauung zu beobachten ist. Die Bewertung der Vergrauung der Proben erfolgt nach der Grauskala DIN 54001 innerhalb 2 h nach Ende der Prüfung (Lagerung bei Raumtemperatur). Die Beurteilungsskala reicht von 5 (sehr gut) bis 1 (sehr schlecht).

### Beispiel 1

100 g natürlicher Glimmerplättchen mit einer Teilchengröße von 10-60 µm werden in 11 Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf 1.8 gehalten wird und 30 Minuten nachgerührt. Eine wässrige Lösung (30 Gew.-%ige Lösung) von 72 g TiCl₄ wird zudosiert, wobei der pH-Wert durch Zugabe von verdünnter Natronlauge auf pH 1.8 gehalten wird. Es wird innerhalb von 6 Stunden so viel Lösung zudosiert, dass das geglühte Pigment 30 Gew.-% TiO₂ enthält und eine silberweiße Interferenz zeigt. Nachdem ca. die Hälfte der TiCl₄-Lösung zudosiert ist, wird eine wässrige Lösung von 9.5 g AlCl₃ x 6H₂O (2 Gew.-%ige Lösung) für die restlichen 3 Stunden parallel zur TiCl₄-Lösung zudosiert. Dann wird der pH-Wert mit verdünnter Salzsäure auf pH 7 eingestellt und innerhalb von einer Stunde eine Lösung aus 6.7 g Zinkchlorid + 1.2 g 37 Gew.-%ige HCl + 80g deionisiertes Wasser zugegeben. Innerhalb von 30 Minuten wird 21 g TEOS (Tetraethoxysilan, 25 Gew.-%ige alkoholische Lösung) zugegeben, 30 Minuten nachgerührt und dann der pH-Wert innerhalb von 60 Minuten auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid, 1.2 Gew.-% ZnO sowie zusätzlich zum Gehalt an Al₂O₃ und SiO₂ im Glimmer + 3 Gew.-% Aluminiumoxid, sowie + 3 Gew.-% Siliciumdioxid. Beispiel 1 stellt ein Interferenzsilber dar, das normalerweise eine Schichtdicke der TiO₂-Schicht pro Seite Substrat von 50-. 60 nm aufweist. Die hier gemessene Schichtdicke der erfindungsgemäßen äußeren Beschichtung beträgt pro Substratseite ca. 90 nm. Das ist eine Erhöhung der Schichtdicke um 50-80%.

### Beispiel 2

100 g natürlicher Glimmerplättchen mit einer Teilchengröße von 10-60 µm werden in 1l Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf pH 1.8 gehalten wird und 30 Minuten nachrührt. Eine wässrige Lösung (30 Gew.-%ige Lösung) von 72 g TiCl₄ wird zudosiert, wobei der pH-Wert durch Zugabe von verdünnter Natronlauge auf pH 1.8 gehalten wird. Es wird innerhalb von 6 Stunden so viel Lösung zudosiert, dass das geglühte Pigment 30 Gew.-% TiO₂ enthält und eine silberweiße Interferenz zeigt. Nachdem die TiCl₄-Lösung zudosiert ist, wird 30 Minuten nachgerührt. Dann wird der pH-Wert mit verdünnter Natronlauge auf pH 7 eingestellt und innerhalb von einer Stunde eine Lösung aus 5 g Zinkchlorid + 0.9 g 37 Gew.-%ige HCl + 60g deionisiertes Wasser zugegeben und 30 Minuten nachgerührt. Dann wird eine wässrige Lösung von 9.5 g AlCl₃ x 6H₂O (2 Gew.-%ige Lösung) über 30 Minuten zudosiert. Anschließend wird 30 Minuten nachgerührt. Bei pH 7 wird innerhalb von 30 Minuten 28 g TEOS (Tetraethoxysilan, 25 Gew.-%ige alkoholische Lösung) zugegeben, 30 Minuten nachgerührt und dann der pH-Wert innerhalb von 60 Minuten auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid, 0.9 Gew.-% ZnO sowie zusätzlich zum Gehalt an Al₂O₃ und SiO₂ im Glimmer + 3 Gew.-% Aluminiumoxid, sowie + 4 Gew.-% Siliciumdioxid. Beispiel 2 stellt ein Interferenzsilber dar, das normalerweise eine Schichtdicke der TiO₂-Schicht pro Seite Substrat von 50 - 60 nm aufweist. Die hier gemessene Schichtdicke der erfindungsgemäßen äußeren Beschichtung beträgt pro Substratseite ca. 90 nm. Das ist eine Erhöhung der Schichtdicke um 50-80%.

### Beispiel 3

100 g natürlicher Glimmerplättchen mit einer Teilchengröße von 10-60 µm werden in 2l Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf 1.8 gehalten wird und 30 Minuten nachgerührt. Eine wässrige Lösung (30 Gew.-%ige Lösung) von 72 g TiCl₄ wird zudosiert, wobei der pH-Wert durch Zugabe von verdünnter Natronlauge auf pH 1.8 gehalten wird. Es wird innerhalb von 6 Stunden so viel Lösung zudosiert, dass das geglühte Pigment 30 Gew.-% TiO₂ enthält und eine silberweiße Interferenz zeigt. Nachdem ungefähr die Hälfte der TiCl₄-Lösung zudosiert ist, werden 100 ml einer wässrigen Lösung von CaCl₂ (14.4 g CaCl₂ x 2 H₂O + 12 ml einer 30%igen H₂O₂-Lösung mit Wasser auf 100 ml aufgefüllt) für die restlichen 3 Stunden parallel zur TiCl₄-Lösung zudosiert und dann 30 Minuten nachgerührt. Anschließend wird der pH-Wert mit verdünnter Natronlauge auf pH 7 eingestellt und innerhalb von einer Stunde eine Lösung aus 5 g Zinkchlorid + 0.9 g 37 Gew.-%ige HCl + 60g deionisiertes Wasser zugegeben und 30 Minuten nachgerührt. Bei pH 7 wird innerhalb von 30 Minuten 28 g TEOS (Tetraethoxysilan, 25 Gew.-%ige alkoholische Lösung) zugegeben, 30 Minuten nachgerührt und dann der pH-Wert innerhalb von 60 Minuten auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid, 0.9 Gew.-% ZnO, 3 Gew.-% Calciumoxid sowie zusätzlich zum Gehalt an SiO₂ im Glimmer + 4 Gew.-% Siliciumdioxid. Beispiel 3 stellt ein Interferenzsilber dar, das normalerweise eine Schichtdicke der TiO₂-Schicht pro Seite Substrat von 50 -60 nm aufweist. Die hier gemessene Schichtdicke der erfindungsgemäßen äußeren Beschichtung beträgt pro Substratseite ca. 90 nm. Das ist eine Erhöhung der Schichtdicke um 50-80%.

### Beispiel 4

100 g synthetische Glimmerplättchen mit einer Teilchengröße von 10-40 µm werden in 2l Wasser suspendiert und unter Rühren auf 75°C erhitzt. Zu der Suspension wird eine wässrige 10 Gew.-%ige Lösung von 3,35 g SnCl₄ innerhalb einer Stunde zudosiert, wobei der pH-Wert durch Zugabe von verdünnter Natronlauge auf 1.8 gehalten wird und 30 Minuten nachgerührt. Eine wässrige Lösung (30 Gew.-%ige Lösung) mit ca. 72 g TiCl₄ wird zudosiert, wobei der pH-Wert durch Zugabe von verdünnter Natronlauge auf pH 1.8 gehalten wird. Es wird innerhalb von 6 Stunden so viel Lösung zudosiert, dass das geglühte Pigment 30 Gew. TiO₂ enthält und eine silberweiße Interferenz zeigt. Nachdem ungefähr die Hälfte der TiCl₄-Lösung zudosiert ist, werden 100 ml einer wässrigen Lösung von CaCl₂ (14,4 g CaCl₂ x 2 H₂O + 12 ml einer 30%igen H₂O₂-Lösung mit Wasser auf 100 ml aufgefüllt) für die restlichen 3 Stunden parallel zur TiCl₄-Lösung zudosiert. Anschließend wird der pH-Wert mit verdünnter Natronlauge auf pH 5 eingestellt und innerhalb von einer Stunde eine Lösung aus 5 g Zinkchlorid + 0,9 g 37 Gew.-%ige HCl + 60g deionisiertes Wasser zugegeben. Bei pH 7 wird innerhalb von 30 Minuten 28 g TEOS (Tetraethoxysilan, 25 Gew.-%ige alkoholische Lösung) zugegeben, 30 Minuten nachgerührt und dann der pH-Wert innerhalb von 60 Minuten auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid, 0,9 Gew. % ZnO sowie zusätzlich zum Gehalt an CaO- und SiO₂-im Glimmer + 1,3% CaO + 4 Gew.-% Siliciumdioxid. Beispiel 4 stellt ein Interferenzsilber dar, das normalerweise eine Schichtdicke der TiO₂-Schicht pro Seite Substrat von 50 -60 nm aufweist. Die hier gemessene Schichtdicke der erfindungsgemäßen äußeren Beschichtung beträgt pro Substratseite ca. 90 nm. Das ist eine Erhöhung der Schichtdicke um 50-80%.

### Beispiel 5

100 g SiO₂-Flakes mit einer Teilchengröße von 10-40 µm werden in 2l Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf 2.2 gehalten wird und 30 Minuten nachgerührt. Eine wässrige Lösung (30 Gew.-%ige Lösung) von 72 g TiCl₄ wird zudosiert, wobei der pH-Wert durch Zugabe von verdünnter Natronlauge auf pH 1.8 gehalten wird. Es wird innerhalb von 6 Stunden so viel Lösung zudosiert, dass das geglühte Pigment 30 Gew.-% TiO₂ enthält und eine silberweiße Interferenz zeigt. Nachdem ungefähr 90% der TiCl₄-Lösung zudosiert ist, wird der pH auf 2.0 eingestellt und die Dosierrate auf die Hälfte verlangsamt. Dann werden parallel zur TiCl₄-Lösung eine Lösung aus 28 g TEOS (Tetraethoxysilan, 12 Gew.-%ige alkoholische Lösung) zudosiert und eine wässrige Lösung aus 9.5 g AlCl₃ x 6H₂O (1 Gew.-%ige Lösung) für die restlichen 1.2 Stunden zugegeben und 10 Minuten nachgerührt. Anschließende wird eine Lösung aus 5 g Zinkchlorid + 0.9 g 37 Gew.-%ige HCl + 60g deionisiertes Wasser zugegeben, 30 Minuten nachgerührt

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid, 0.9 Gew.-% ZnO, 3 Gew.-% Aluminiumoxid sowie zusätzlich zum Gehalt an SiO₂ im Glimmer + 4 Gew.-% Siliciumdioxid. Das Beispiel 5 stellt ein Interferenzsilber dar, das normalerweise eine Schichtdicke der TiO₂-Schicht pro Seite Substrat von 50 - 60 nm aufweist. Die hier gemessene Schichtdicke der erfindungsgemäßen äußeren Beschichtung beträgt pro Substratseite ca. 90 nm. Das ist eine Erhöhung der Schichtdicke um 50-80%.

### Beispiel 6 (Referenz I)

100 g Glimmerplättchen mit einer Teilchengröße von 10-60 µm werden in 1l Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf 1.8 gehalten wird und 30 Minuten nachgerührt. Eine wässrige Lösung (30 Gew.-%ige Lösung) von 72 g TiCl₄ wird innerhalb von 6 Stunden zudosiert, wobei der pH-Wert durch Zugabe von verdünnter Natronlauge auf 1.8 gehalten wird. Anschließend wird 30 Minuten nachgerührt. Dann wird der pH-Wert mit verdünnter Salzsäure auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid. Beispiel 6 stellt ein Interferenzsilber dar, das eine Schichtdicke der TiO₂-Schicht pro Seite Substrat von ca. 50 nm aufweist.

### Beispiel 7 (Referenz II)

100 g Glimmerplättchen mit einer Teilchengröße von 10-60 µm werden in 1l Wasser suspendiert und unter Rühren auf 75°C erhitzt. Zu der Suspension wird eine wässrige 10 Gew.-%ige Lösung von 3.35 g SnCl₄ innerhalb einer Stunde zudosiert, wobei der pH-Wert durch Zugabe von verdünnter Natronlauge auf 1.8 gehalten wird und 30 Minuten nachgerührt. Eine wässrige Lösung (30 Gew.-%ige Lösung) von 72g TiCl₄ wird innerhalb von 6 Stunden zudosiert, wobei der pH-Wert durch Zugabe von verdünnter Natronlauge auf 1.8 gehalten wird. Nachdem die Hälfte der TiCl₄-Lösung, wird eine wässrige Lösung von 9.5 g AlCl₃ x 6H₂O für die restlichen 3 Stunden parallel zur TiCl₄-Lösung zudosiert. Anschließend wird 30 Minuten nachgerührt. Dann wird der pH-Wert mit verdünnter Salzsäure auf pH 6 eingestellt.

Die Suspension wird aufgearbeitet. Das Pigment wird abfiltriert, gewaschen, getrocknet, bei 900°C calciniert und anschließend gesiebt. Das calcinierte Pigment enthält 30 Gew.-% Titandioxid. Beispiel 7 stellt ein Interferenzsilber dar, das eine Schichtdicke der TiO₂-Schicht pro Seite Substrat von ca. 50 nm aufweist.

Die Ergebnisse der Testversuche sind in Tabelle 1 dargestellt. Die Pigmente der erfindungsgemäßen Beispiele 1 und 2 zeigen eine deutlich bessere Stabilität und eine geringere Porosität als ein Pigment des Standes der Technik. Zudem ist die BET-Oberfläche (gemessen nach DIN ISO 9277: 2003-05) in Beispiel 1 um 63% und in Beispiel 2 um 69% im Vergleich mit Referenz I (Beispiel 6) erniedrigt.

**Tabelle 1: Beispiele für Glimmer der Teilchengröße 10-60 µm bzw. 10-40 µm**

| Beispiel | BET (Glühung bei 900°C) | Lichtstabilität | Photoaktivität | Vergilbung |
|---|---|---|---|---|
| Referenz I | 6,5 m²/g | 6 | 2-1 | 1 |
| Referenz II | 4,2 m²/g | 6-7 | 2-1 | 1 |
| 1 | 2,4 | 8 | 4 | 3 |
| 2 | 2,0 | 8 | 3 | 4 |
| 3 | 2,3 | 8 | 3 | 4 |
| 4 | 3,0 | 8 | 3 | 4 |

## Patentansprüche

1. Effektpigmente auf der Basis unbeschichteter, plättchenförmiger Substrate oder mit ein oder mehreren Metalloxiden beschichteter, plättchenförmiger Substrate **dadurch gekennzeichnet, dass** sie eine äußere metalloxidhaltige, calcinierte Beschichtung umfassen, welche aus a) TiO₂ und b) Al₂O₃, MgO und/oder CaO, und c) SiO₂ und d) ZnO und/oder e) mindestens ein Mischoxid der unter a) bis d) genannten Elemente besteht, wobei das Effektpigmente eine Interferenzfarbe aufweist, die äußere Beschichtung dicker ist als sie es für die Erreichung der Interferenzfarbe physikalisch und theoretisch wäre und die Dicke der äußeren Beschichtung ≥20% mehr beträgt als die physikalische Schichtdicke der Interferenzfarbe.

2. Effektpigmente nach Anspruch 1, **dadurch gekennzeichnet, dass** die äußere Beschichtung aus a) TiO₂ und b) Al₂O₃, MgO und/oder CaO, und c) SiO₂ und d) ZnO und/oder mindestens einem Mischoxid dieser Elemente besteht, wobei die Beschichtung die Al-, Mg-, Ca-, Si- und Zn-Oxide und/oder -mischoxide vorwiegend im äußeren Bereich enthält.

3. Effektpigmente nach einem oder mehreren der Ansprüche 1 bis 2,
**dadurch gekennzeichnet, dass** die äußere Beschichtung aus TiO₂, Al₂O₃, SiO₂ und ZnO und/oder mindestens einem Mischoxid dieser Elemente besteht, wobei die Beschichtung die Al-, Si- und Zn-Oxide und/oder - mischoxide vorwiegend im äußeren Bereich enthält.

4. Effektpigmente nach einem oder mehreren der Ansprüche 1 bis 3,
**dadurch gekennzeichnet, dass** sie in der äußeren Beschichtung ≥ 20 Gew.-% TiO₂, ≥ 0.5 Gew.-% ZnO, ≥ 0.1 Gew.-% Al₂O₃ und ≥ 1.5 Gew.-% SiO₂ enthalten, wobei die Gew.-% auf das Gesamtgewicht des geglühten Pigments bezogen sind.

5. Effektpigmente nach einem oder mehreren der Ansprüche 1 bis 4
**dadurch gekennzeichnet, dass** sie eine spezifische Oberfläche aufweisen, die gegenüber der spezifischen Oberfläche eines eine geglühte TiO₂-Schicht als äußere Schicht enthaltenden Pigments um 30 - 80 % reduziert ist.

6. Effektpigmente nach einem oder mehreren der Ansprüche 1 bis 5
**dadurch gekennzeichnet, dass** die plättchenförmigen Substrate ausgewählt sind aus Plättchen aus synthetischem oder natürlichem Glimmer, Glasplättchen, SiO₂-Plättchen und Al₂O₃-Plättchen, insbesondere aus Plättchen aus synthetischem oder natürlichem Glimmer.

7. Effektpigmente nach einem oder mehreren der Ansprüche 1 bis 6,
**dadurch gekennzeichnet, dass** die plättchenförmigen Substrate mit Oxiden und/oder Oxidhydraten des Aluminiums, Siliziums, Eisens, Zinns und Titans, insbesondere Titandioxid, in der Rutil- oder Anatas-Modifikation, und Gemischen dieser Verbindungen beschichtet sind.

8. Effektpigmente nach einem oder mehreren der Ansprüche 1 bis 7,
**dadurch gekennzeichnet, dass** die äußere Beschichtung des geglühten Pigments eine Dicke von ≥ 50 nm hat.

9. Effektpigmente nach einem oder mehreren der Ansprüche 1 bis 8,
**dadurch gekennzeichnet, dass** die Dicke der äußeren Beschichtung ≥40% mehr beträgt als die physikalische Schichtdicke der entsprechenden Interferenzfarbe.

10. Verfahren zur Herstellung der Effektpigmente nach einem oder mehreren der Ansprüche 1 bis 9, **dadurch gekennzeichnet, dass** a) unbeschichtete, plättchenförmige Substrate oder mit ein oder mehreren Metalloxiden beschichtete, plättchenförmige Substrate mittels eines nass-chemischen Verfahren mit Ti- und Zn- und Al-, Mg- und/oder Ca-, und Si-oxiden, -hydroxiden und/oder -oxidhydraten beschichtet werden und b) die so beschichteten plättchenförmigen Substrate aufgearbeitet und anschließend calciniert werden.

11. Verfahren nach Anspruch 10, **dadurch gekennzeichnet, dass** die Substrate in einer wässrigen Lösung suspendiert werden, Ti-, Si-, Zn- und Al-, bzw. Al-, Mg- und/oder oder Ca-, -Salzlösungen bei einem für die Hydrolyse der Salze geeigneten pH-Wert zugegeben werden und wobei der pH-Wert so ausgewählt ist, dass die Metalloxide bzw. Hydroxide bzw. Oxidhydrate auf den Substraten abgeschieden werden.

12. Verfahren nach einem oder mehreren der Ansprüche 10 bis 11, **dadurch gekennzeichnet, dass** die Ti- und die Al-, bzw. Al-, Mg- und/oder Ca-, - Salzlösungen gleichzeitig zudosiert werden, wobei ein Teil der Ti-Salzlösung auch zu Anfang allein zugegeben werden kann.

13. Verfahren nach einem oder mehreren der Ansprüche 10 bis 12, **dadurch gekennzeichnet, dass** zuerst die Zn-Salzlösung, dann die Al-Salzlösung und dann die Si-Salzlösung zugegeben werden.

14. Verfahren nach einem oder mehreren der Ansprüche 10 bis 13, **dadurch gekennzeichnet, dass** die Zugabe der Si- und Al-Salzlösungen gleichzeitig erfolgt.

15. Effektpigmente hergestellt nach einem Verfahren gemäß einem oder mehreren der Ansprüche 10 bis 14.

16. Verwendung der Effektpigmente nach einem oder mehreren der Ansprüche 1 bis 9 in Farben, Lacken, Automobillacken, Pulverlacken, Druckfarben, Sicherheitsdruckfarben, Kunststoffen, keramischen Materialien, Gläsern, Papier, im Papierstrich, in Tonern für elektrophotographische Druckverfahren, im Saatgut, in Gewächshausfolien und Zeltplanen, als Absorber bei der Lasermarkierung von Papier und Kunststoffen, als Absorber beim Laserschweißen von Kunststoffen, kosmetischen Formulierungen, zur Herstellung von Pigmentanteigungen mit Wasser, organischen und/oder wässrigen Lösemitteln, zur Herstellung von Pigmentpräparationen und Trockenpräparaten.

## Claims

1. Effect pigments based on uncoated, flake-form substrates or flake-form substrates coated with one or more metal oxides, **characterised in that** they comprise an outer metal oxide-containing, calcined coating which consists of a) TiO₂ and b) Al₂O₃, MgO and/or CaO, and c) SiO₂ and d) ZnO and/ or e) at least one mixed oxide of the elements mentioned under a) to d), where the effect pigment has an interference colour, the outer coating is thicker than it would physically and theoretically be for achieving the interference colour and the thickness of the outer coating is ≥ 20% more than the physical layer thickness of the interference colour.

2. Effect pigments according to Claim 1, **characterised in that** the outer coating consists of a) TiO₂ and b) Al₂O₃, MgO and/or CaO, and c) SiO₂ and d) ZnO and/or at least one mixed oxide of these elements, where the coating comprises the Al, Mg, Ca, Si and Zn oxides and/or mixed oxides predominantly in the outer region.

3. Effect pigments according to one or more of Claims 1 to 2, **characterised in that** the outer coating consists of TiO₂, Al₂O₃, SiO₂ and ZnO and/or at least one mixed oxide of these elements, where the coating comprises the Al, Si and Zn oxides and/or mixed oxides predominantly in the outer region.

4. Effect pigments according to one or more of Claims 1 to 3, **characterised in that** they comprise ≥ 20% by weight of TiO₂, ≥ 0.5% by weight of ZnO, ≥ 0.1% by weight of Al₂O₃ and ≥ 1.5% by weight of SiO₂ in the outer coating, where the % by weight are based on the total weight of the calcined pigment.

5. Effect pigments according to one or more of Claims 1 to 4, **characterised in that** they have a specific surface area which is reduced by 30 - 80% compared with the specific surface area of a pigment comprising a calcined TiO₂ layer as outer layer.

6. Effect pigments according to one or more of Claims 1 to 5, **characterised in that** the flake-form substrates are selected from synthetic or natural mica flakes, glass flakes, SiO₂ flakes and Al₂O₃ flakes, in particular from synthetic or natural mica flakes.

7. Effect pigments according to one or more of Claims 1 to 6, **characterised in that** the flake-form substrates are coated with oxides and/or oxide hydrates of aluminium, silicon, iron, tin and titanium, in particular titanium dioxide, in the rutile or anatase modification, and mixtures of these compounds.

8. Effect pigments according to one or more of Claims 1 to 7, **characterised in that** the outer coating of the calcined pigment has a thickness of ≥ 50 nm.

9. Effect pigments according to one or more of Claims 1 to 8, **characterised in that** the thickness of the outer coating is ≥ 40% more than the physical layer thickness of the corresponding interference colour.

10. Process for the preparation of the effect pigments according to one or more of Claims 1 to 9, **characterised in that** a) uncoated, flake-form substrates or flake-form substrates coated with one or more metal oxides are coated with oxides, hydroxides and/or oxide hydrates of Ti and Zn and Al, Mg and/or Ca, and Si by means of a wet-chemical process, and b) the flake-form substrates coated in this way are worked up and subsequently calcined.

11. Process according to Claim 10, **characterised in that** the substrates are suspended in an aqueous solution, Ti, Si, Zn and Al, or Al, Mg and/or Ca salt solutions are added at a pH which is suitable for hydrolysis of the salts, and where the pH is selected so that the metal oxides or hydroxides or oxide hydrates are deposited on the substrates.

12. Process according to one or more of Claims 10 to 11, **characterised in that** the Ti salt solution and the Al or Al, Mg and/or Ca salt solution are metered in simultaneously, where some of the Ti salt solution may also be added alone at the beginning.

13. Process according to one or more of Claims 10 to 12, **characterised in that** firstly the Zn salt solution, then the Al salt solution and then the Si salt solution are added.

14. Process according to one or more of Claims 10 to 13, **characterised in that** the Si and Al salt solutions are added simultaneously.

15. Effect pigments prepared by a process according to one or more of Claims 10 to 14.

16. Use of the effect pigments according to one or more of Claims 1 to 9 in paints, coatings, automotive paints, powder coatings, printing inks, security printing inks, plastics, ceramic materials, glasses, paper, in paper coating, in toners for electrophotographic printing processes, in seed, in greenhouse sheeting and tarpaulins, as absorbers in the laser marking of paper and plastics, as absorbers in the laser welding of plastics, cosmetic formulations, for the preparation of pigment pastes with water, organic and/or aqueous solvents, for the preparation of pigment preparations and dry preparations.

## Revendications

1. Pigments à effet basés sur des substrats sous forme de flocons non revêtus ou des substrats sous forme de flocons revêtus d'un ou de plusieurs oxyde(s) métallique(s), **caractérisés en ce qu'**ils comprennent un revêtement externe calciné contenant un oxyde métallique qui est constitué par a) du TiO₂ et b) de l'Al₂O₃, du MgO et/ou du CaO, et c) du SiO₂ et d) du ZnO et/ou e) par au moins un oxyde mixte des éléments mentionnés sous a) à d), où le pigment à effet présente une couleur d'interférence, le revêtement externe est plus épais qu'il devrait être physiquement et théoriquement pour obtenir la couleur d'interférence et l'épaisseur du revêtement externe est supérieure de 20% ou plus à l'épaisseur de couche physique de la couleur d'interférence.

2. Pigments à effet selon la revendication 1, **caractérisés en ce que** le revêtement externe est constitué par a) du TiO₂ et b) de l'Al₂O₃, du MgO et/ou du CaO, et c) du SiO₂ et d) du ZnO et/ou par au moins un oxyde mixte de ces éléments, où le revêtement comprend des oxydes d'Al, de Mg, de Ca, de Si et de Zn et/ou des oxydes mixtes de façon prédominante dans la région externe.

3. Pigments à effet selon une ou plusieurs des revendications 1 et 2, **caractérisés en ce que** le revêtement externe est constitué par du TiO₂, de l'Al₂O₃, du SiO₂ et du ZnO et/ou par au moins un oxyde mixte de ces éléments, où le revêtement comprend les oxydes d'Al, de Si et de Zn et/ou les oxydes mixtes de façon prédominante dans la région externe.

4. Pigments à effet selon une ou plusieurs des revendications 1 à 3, **caractérisés en ce qu'**ils comprennent une quantité ≥ 20% en poids de TiO₂, ≥ 0,5% en poids de ZnO, ≥ 0,1% en poids d'Al₂O₃ et ≥ 1,5% en poids de SiO₂ dans le revêtement externe, où les % en poids sont basés sur le poids total du pigment calciné.

5. Pigments à effet selon une ou plusieurs des revendications 1 à 4, **caractérisés en ce qu'**ils présentent une aire de surface spécifique qui est réduite de 30 - 80% par comparaison avec l'aire de surface spécifique d'un pigment comprenant une couche en TiO₂ calciné en tant que couche externe.

6. Pigments à effet selon une ou plusieurs des revendications 1 à 5, **caractérisés en ce que** les substrats sous forme de flocons sont choisis parmi des flocons de mica synthétique ou naturel, des flocons de verre, des flocons de SiO₂ et des flocons d'Al₂O₃, en particulier parmi des flocons de mica synthétique ou naturel.

7. Pigments à effet selon une ou plusieurs des revendications 1 à 6, **caractérisés en ce que** les substrats sous forme de flocons sont revêtus d'oxydes et/ou d'hydrates d'oxydes d'aluminium, de silicium, de fer, d'étain et de titane, en particulier de dioxyde de titane, selon la modification rutile ou anatase, et de mélanges de ces composés.

8. Pigments à effet selon une ou plusieurs des revendications 1 à 7, **caractérisés en ce que** le revêtement externe du pigment calciné présente une épaisseur ≥ 50 nm.

9. Pigments à effet selon une ou plusieurs des revendications 1 à 8, **caractérisés en ce que** l'épaisseur du revêtement externe est supérieure de 40% ou plus à l'épaisseur de couche physique de la couleur d'interférence correspondante.

10. Procédé pour la préparation des pigments à effet selon une ou plusieurs des revendications 1 à 9, **caractérisé en ce que** a) des substrats sous forme de flocons non revêtus ou des substrats sous forme de flocons revêtus d'un ou de plusieurs oxyde(s) métallique(s) sont revêtus d'oxydes, d'hydroxydes et/ou d'hydrates d'oxydes de Ti et de Zn et d'Al, de Mg et/ou de Ca, et de Si au moyen d'un procédé chimique par voie humide, et b) les substrats sous forme de flocons revêtus de cette façon sont élaborés et sont ensuite calcinés.

11. Procédé selon la revendication 10, **caractérisé en ce que** les substrats sont suspendus dans une solution aqueuse, des solutions de sels de Ti, de Si, de Zn et d'Al, ou d'Al, de Mg et/ou de Ca sont ajoutées à un pH qui convient pour l'hydrolyse des sels, et où le pH est choisi de telle sorte que les oxydes ou hydroxydes ou hydrates d'oxydes métalliques soient déposés sur les substrats.

12. Procédé selon une ou plusieurs des revendications 10 et 11, **caractérisé en ce que** la solution de sel de Ti et la solution de sel d'Al ou d'Al, de Mg et/ou la solution de sel de Ca sont mesurées simultanément, où une certaine part de la solution de sel de Ti peut également être ajoutée seule au début.

13. Procédé selon une ou plusieurs des revendications 10 à 12, **caractérisé en ce que**, en premier lieu, la solution de sel de Zn, puis la solution de sel d'Al puis la solution de sel de Si sont ajoutées.

14. Procédé selon une ou plusieurs des revendications 10 à 13, caractérisé les solutions de sels de Si et d'Al sont ajoutées simultanément.

15. Pigments à effet préparés au moyen d'un procédé selon une ou plusieurs des revendications 10 à 14.

16. Utilisation des pigments à effet selon une ou plusieurs des revendications 1 à 9 dans des peintures, des revêtements, des peintures d'automobiles, des revêtements pulvérulents, des encres d'impression, des encres d'impression de sécurité, des matières plastiques, des matériaux de céramique, des verres, du papier, au niveau du couchage du papier, dans des toners pour des processus d'impression électrophotographique, au niveau des semences, au niveau de feuilles pour serres et de bâches, en tant qu'absorbeurs au niveau du marquage laser du papier et des matières plastiques, en tant qu'absorbeurs au niveau du soudage laser de matières plastiques, dans des formulations cosmétiques, pour la préparation de pâtes de pigments avec de l'eau, des solvants organiques et/ou aqueux, pour la préparation de préparations de pigments et de préparations sèches.
